(19) Europäisches Patentamt
European Patent Office
Office européen des brevets

(11) Veröffentlichungsnummer: **0 105 243**
**B1**

# (12) EUROPÄISCHE PATENTSCHRIFT

(45) Veröffentlichungstag der Patentschrift: **03.08.88**

(51) Int. Cl.⁴: **C 07 D 211/22** // A61K31/445

(21) Anmeldenummer: **83109114.5**

(22) Anmeldetag: **15.09.83**

(54) **Spaltung von rac. 1-Propyl-3-aryl-piperidinen.**

(30) Priorität: **01.10.82 CH 5809/82**

(43) Veröffentlichungstag der Anmeldung: **11.04.84 Patentblatt 84/15**

(45) Bekanntmachung des Hinweises auf die Patenterteilung: **03.08.88 Patentblatt 88/31**

(84) Benannte Vertragsstaaten: **AT BE CH DE FR GB IT LI LU NL SE**

(56) Entgegenhaltungen:
**EP - A - 0 030 526**

**TETRAHEDRON LETTERS, Band 24, Nr. 4, 1983, Seiten 343-346, Pergamon Press, Oxford, GB; W. ARNOLD u.a.: "An efficient resolution of 3-ppp and assignment of the absolute configuration"**

(73) Patentinhaber: **F. HOFFMANN-LA ROCHE & CO. Aktiengesellschaft, CH-4002 Basel (CH)**

(72) Erfinder: **Imhof, René, Dr., Bleumatthöhe 3, CH-5264 Gipf-Oberfrick (CH)**

(74) Vertreter: **Kellenberger, Marcus, Dr. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 105 243 B1

**Beschreibung**

Die vorliegende Erfindung betrifft ein neues Verfahren zur Herstellung der beiden Enantiomeren von 3-(1-Propyl-3-piperidinyl)phenol (3-PPP) der Formel

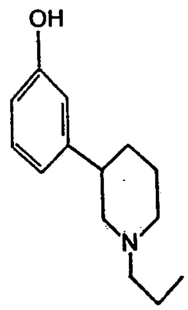

I

d.h. von (+)-3-PPP und (−)-3-PPP.

Die racemische Verbindung 3-PPP der obigen Formel I, deren Herstellung sowie deren ausserordentlich interessante pharmakologische Wirkung als selektiver, dopaminerger Autorezeptoragonist wurden von A. Carlsson et al. in Life Sciences 28, 1225 (1981) und J. Med. Chem. 24, 1475 (1981) sowie in EP-A-30 526 beschrieben. Wie anlässlich des Symposium on Dopamine Receptor Agonists vom 20.–23. April 1982 in Stockholm bekannt wurde, gelang es der gleichen Forschungsgruppe in der Folge auch, die beiden Enantiomeren (+)-3-PPP und (−)-3-PPP herzustellen und separat zu testen, wobei beide Enantiomere als interessant befunden wurden. Die beiden Enantiomeren wurden nicht durch Racematspaltung von racemischem 3-PPP hergestellt, sondern durch Auftrennung von racemischem 3-(3-Methoxyphenyl)-1-benzylpiperidin, dessen beide Enantiomeren dann in mehreren Schritten in die erwünschten Endprodukte überführt wurden. Die Tatsache, dass die Racematspaltung auf einer relativ frühen Stufe der Synthese erfolgt, hat zur Folge, dass die Herstellung der beiden Enantiomeren (+)-3-PPP und (−)-3-PPP ausserordentlich arbeitsaufwendig wird, da die restlichen Stufen der Synthese jeweils doppelspurig geführt werden müssen. Die mehrstufige Synthese zur Herstellung der beiden Enantiomeren ist im nachfolgenden Reaktionsschema formelmässig skizziert.

**Schema**

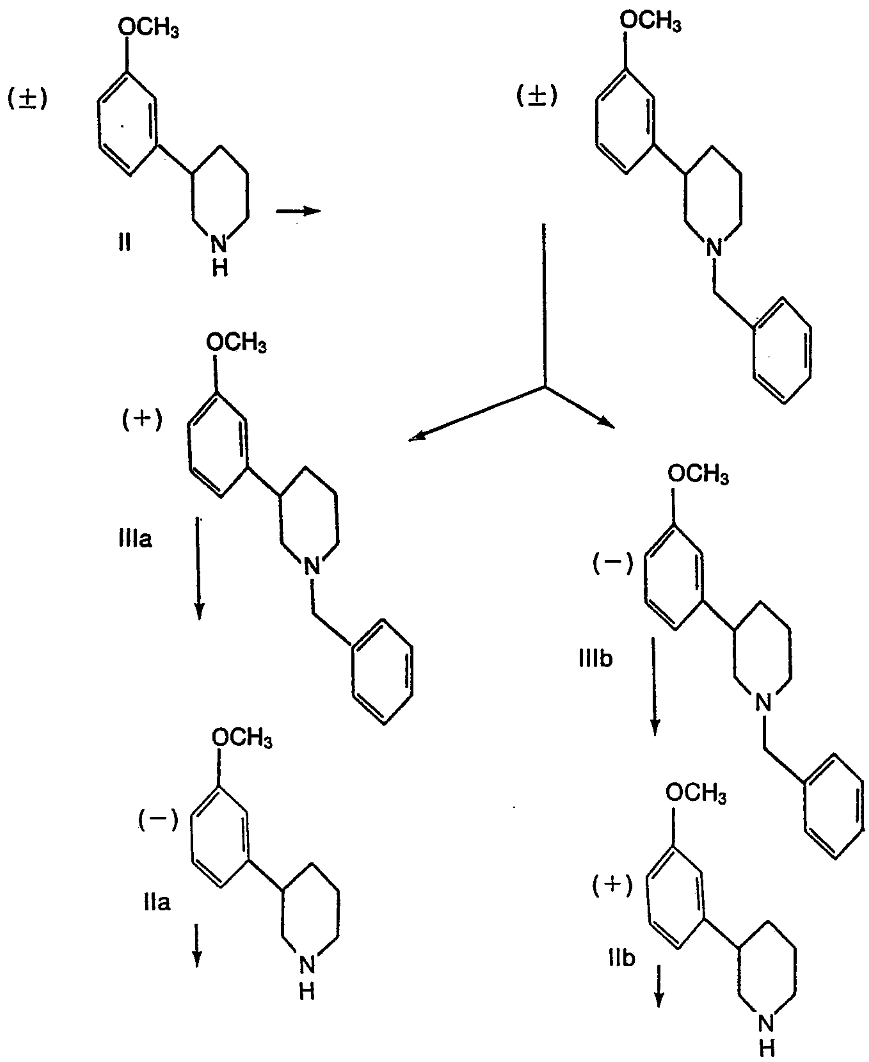

(±) II, (±) III, (+) IIIa, (−) IIIb, (−) IIa, (+) IIb

(+)

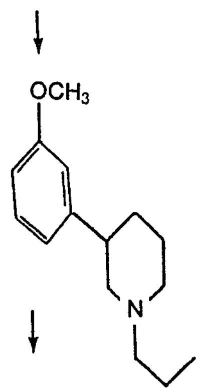

IVa

(−)

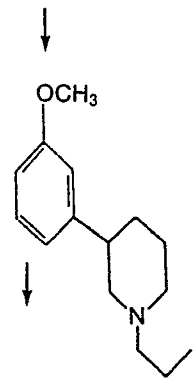

IVb

(+)

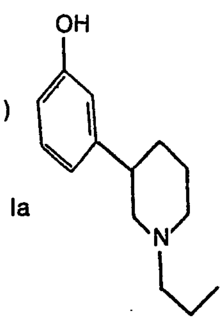

Ia

(−)

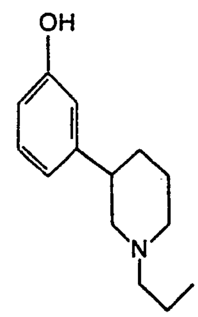

Ib

In der ersten Stufe wurde das racemische 3-(3-Methoxyphenyl)piperidin der Formel II mit Benzylchlorid in das entsprechende N-Benzylderivat III übergeführt, welches dann mit Hilfe von optisch aktiver Dibenzoylweinsäure in die beiden Enantiomeren IIIa und IIIb aufgetrennt wurde. Danach wurden die beiden Enantiomeren IIIa und IIIb mittels Wasserstoff/Palladium zu den entsprechenden Enantiomeren IIa und IIb debenzyliert, welche danach unter Bildung der Enantiomeren IVa und IVb am Stickstoffatom propyliert wurden. Durch Ätherspaltung mit Bromwasserstoff wurden schliesslich die erwünschten Enantiomeren Ia und Ib erhalten.

Da die oben skizzierte Synthese wegen des grossen Arbeitsaufwandes wohl nicht zu genügen weiss, wurde in Trends Pharmacol. Sci. 3, 322 (1982) die Racematspaltung von 3-PPP von der Forschungsgruppe um A. Carlsson als wünschenswert bezeichnet, obwohl sie ihnen bisher offenbar nicht gelungen war.

Im Rahmen der vorliegenden Erfindung wurde nun überraschenderweise festgestellt, dass sich racemisches 3-PPP sowie dessen entsprechender Methoxyäther mit optisch aktiver 2,2′-(1,1′-Binaphthyl)phosphorsäure in einfacher Weise in die entsprechenden Enantiomeren aufspalten lassen. Demnach betrifft die vorliegende Erfindung ein Verfahren zur Herstellung der beiden Enantiomeren (+)-3-PPP und (−)-3-PPP, welches dadurch gekennzeichnet ist, dass man eine racemische Verbindung der allgemeinen Formel

(±)

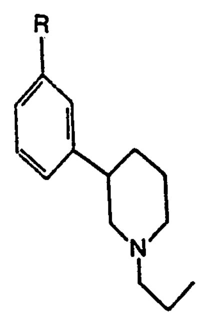

V

worin R Hydroxy oder Methoxy bedeutet, mit optisch aktiver 2,2′-(1,1′-Binaphthyl)phosphorsäure in die beiden Enantiomeren auftrennt und nötigenfall die Methoxygruppe in 3-Stellung abspaltet.

Die Racematspaltung erfolgt nach hierfür an sich üblichen Methoden, d.h. durch Umsetzen des Racemates mit optisch aktiver 2,2′-(1,1′-Binaphthyl)phosphorsäure, Trennung der erhaltenen diastereoisomeren Salze, z.B. durch fraktionierte Kristallisation, und anschliessendem Freisetzen der optisch einheitlichen Base. Die Umsetzung mit der optisch aktiven Säure erfolgt in einem unter den Reaktionsbedingungen inerten Lösungsmittel oder Lösungsmittelgemisch, in welchem das racemische Ausgangsmaterial löslich ist, wobei die fraktionierte Kristallisation vorzugsweise im gleichen Lösungsmittel oder Lösungsmittelgemisch erfolgt. Geeignete Lösungsmittel sind Alkohole,

wie Methanol oder Äthanol, chlorierte Kohlenwasserstoffe, wie Methylenchlorid, Essigester und dgl., wobei ein Gemisch von Methanol und Methylenchlorid besonders bevorzugt ist.

Die Abspaltung der Methoxygruppe erfolgt ebenfalls nach an sich üblichen Methoden mit Bromwasserstoff, beispielsweise durch einfaches Kochen des Eduktes in 48%-iger Bromwasserstoffsäure.

Die folgenden Beispiele veranschaulichen die Erfindung. Alle Temperaturen sind in Grad Celsius angegeben.

Beispiel 1

Herstellung von (−)-3-PPP

Eine Lösung von 5,8 g (0,026 Mol) 3-PPP in 30 ml Methanol werden mit 6,3 g (0,018 Mol) (+)-2,2'-(1,1'-Binaphthyl)phosphorsäure in 200 ml Methanol und 20 ml Methylenchloid versetzt. Dieses Gemisch wird am Rotationsverdampfer unter vermindertem Druck auf ca. 100 ml Volumen eingedampft, worauf Kristallisation einsetzt. Nach Stehenlassen über Nacht im Kühlschrank wird der Kristallbrei abgenutscht und mit 2 × 50 ml kaltem Methanol/Essigester (1:1) gewaschen. Das erhaltene Kristallisat wird aus 100 ml heissem Methanol umkristallisiert, erneut über Nacht im Kühlschrank stehen gelassen und dann abgenutscht und mit 2 × 25 ml kaltem Methanol/Essigester gewaschen. Aus dem erhaltenen Binaphthylphosphorsäuresalz Smp. 271–273° kann die freie Base von 3-PPP durch Verteilen zwischen Essigester und wässrigem Ammoniak aus der organischen Phase isoliert werden. Nach Filtration über 80 g Kieselgel mit Essigester als Eluierungsmittel werden 2,1 g (72,5%) (−)-3-PPP erhalten. Dieses wird mit Chlorwasserstoff in Alkohol/Diäthyläther ins Hydrochlorid überführt: 2,3 g (−)-3-PPP HCl, Smp. 185–186°, $[\alpha]^{20}_{589} = -7{,}4°$ (c = 2,2 in Methanol).

Beispiel 2

Herstellung von (+)-3-PPP

6,8 g (0,031 Mol) mit (+)-Enantiomerem angereicherte Base 3-PPP (erhalten nach der Abtrennung des (−)-Enantiomeren) wird in 30 ml Methanol gelöst und mit 6,8 g (0,02 Mol) (−)-2,2'-(1,1'-Binaphthyl)phosphorsäure in 200 ml Methanol und 50 ml Methylenchlorid versetzt. Beim Eindampfen dieses Gemisches am Rotationsverdampfer unter vermindertem Druck auf die Hälfte des Volumens setzt Kristallisation ein, die im Kühlschrank über Nacht vervollständigt wird. Der Kristallbrei wird abgenutscht und mit 2 × 50 ml kaltem Methanol/Essigester (1:1) gewaschen. Darauf löst man die Kristalle erneut in 100 ml heissem Methanol und kristallisiert erneut im Kühlschrank über Nacht. Aus dem erhaltenen Binaphthylphosphorsäuresalz Smp. 271–273° kann die Base von 3-PPP durch Verteilen zwischen Essigester und wässrigem Ammoniak aus der organischen Phase isoliert werden. Nach Filtration über 80 g Kieselgel mit Essigester als Eluierungsmittel werden 3,4 g (ca. 75%) (+)-3-PPP erhalten. Diese werden mit Chlorwasserstoff in Alkohol/Diäthyläther ins Hydrochlorid übergeführt. 3,4 g (+)-3-PPP HCl, Smp. 184–185°, $[\alpha]^{20}_{589} = +7{,}4°$ (c = 2,2 in Methanol).

Beispiel 3

Herstellung von (−)-3-(3-Methoxyphenyl)-1-propylpiperidin

Zu einer Lösung von 2,6 g (0,011 Mol) 3-(3-Methoxyphenyl)-1-propylpiperidin in 20 ml Methylenchlorid werden 2,1 g (0,006 Mol) (+)-2,2'-(1,1'-Binaphthyl)phosphorsäure in 50 ml Methylenchlorid/Methanol (2:1) gegeben, und das Gemisch anschliessend am Rotationsverdampfer unter vermindertem Druck auf ca. 50 ml Volumen eingeengt, wobei Kristallisation einsetzt. Nach Stehenlassen über Nacht im Kühlschrank wird der Kristallbrei abgenutscht und mit 2 × 25 ml kaltem Methanol/Essigester (1:1) gewaschen. Man erhält 1,95 g weisse Kristalle, welche in 20 ml heissem Methanol gelöst und im Kühlschrank wieder kristallisiert werden. Es verbleiben 1,5 g Binaphthylphosphorsäuresalz Smp. 216–218°, aus welchem die freie Base durch Verteilen zwischen Essigester und wässrigem Ammoniak aus der organischen Phase isoliert werden kann: 0,9 g als Öl. Nach Filtration über 30 g Kieselgel mit Essigester als Eluierungsmittel erhält man 0,75 g (58%) (−)-3-(3-Methoxyphenyl)-1-propylpiperidin, $[\alpha]^{20}_{589} = -10{,}9°$ (c = 1 in Methanol), Schmelzpunkt des Hydrobromidsalzes 190–191°.

Beispiel 4

Herstellung von (+)-3-(3-Methoxyphenyl)-1-propylpiperidin

4,0 g (0,017 Mol) mit (+)-Enantiomerem angereicherte Base von 3-(3-Methoxyphenyl)-1-propylpiperidin (erhalten nach der Abtrennung des (−)-Enatiomeren) werden in 35 ml Methylenchlorid gelöst und mit 6,0 g (0,017 Mol) (−)-2,2'-(1,1'-Binaphthyl)phosphorsäure in 150 ml Methylenchlorid/Methanol (2:1) versetzt. Beim Eindampfen auf ca. 100 ml Volumen setzt Kristallisation ein. Der erhaltene Kristallbrei wird nach Stehenlassen über Nacht im Kühlschrank abgenutscht und mit 2 × 30 ml kaltem Methanol/Essigester (1:1) gewaschen. Man erhält 4 g Binaphthylphosphorsäuresalz, welches aus 50 ml Methanol umkristallisiert wird. Die abgenutschten und getrockneten Kristalle Smp. 216–218° (3,25 g) werden zwischen Essigester und wässrigem Ammoniak verteilt und so aus der organischen Phase 1.35 g (+)-3-(3-Methoxyphenyl)-1-propylpiperidin isoliert. Diese werden über eine 50 g Kieselgelsäule mit Essigester filtriert, wobei man 1,1 g (+)-3-(3-Methoxyphenyl)-1-propylpiperidin erhält, $[\alpha]^{20}_{589} = +11{,}2°$ (c = 1 in Methanol), Schmelzpunkt des Hydrobromidsalzes 190–191°.

Beispiel 5

Herstellung von (−)-3-PPP aus (−)-3-(3-Methoxyphenyl)-1-propylpiperidin

0,45 g (0,002 Mol) (−)-3-(3-Methoxyphenyl)-1-propylpiperidin werden mit 10 ml 48%-iger Bromwasserstoffsäure während 1 Stunde erhitzt. Anschliessend entfernt man das Lösungsmittel am Rotationsverdampfer unter vermindertem Druck und verteilt den Rückstand zwischen Methylenchlorid und gesättigter wässriger Bicarbonatlösung. Das aus der organischen Phase isolierte (−)-3-PPP wird über eine 20 g Kieselgelsäule filtriert: 0,35 g (80%); Hydrochloridsalz aus Alkohol/Diäthyläther, Smp. 180–181°, $[\alpha]^{20}_{589} = -7,5°$ (c = 2,2 in Methanol).

**Patentansprüche**

1. Verfahren zur Herstellung der beiden Enantiomeren von 3-(1-Propyl-3-piperidinyl)phenol der Formel

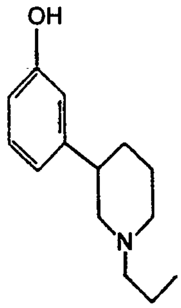

I

dadurch gekennzeichnet, dass man eine racemische Verbindung der allgemeinen Formel

(±)

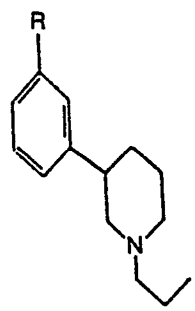

V

worin R Hydroxy oder Methoxy bedeutet, mit optisch aktiver 2,2′-(1,1′-Binaphthyl)phosphorsäure in die beiden Enantiomeren auftrennt und nötigenfalls die Methoxygruppe in 3-Stellung abspaltet.

2. Verfahren nach Anspruch 1, dadurch gekennzeichnet, dass man racemisches 3-(1-Propyl-3-piperidinyl)phenol mit optisch aktiver 2,2′-(1,1′-Binaphthyl)phosphorsäure umsetzt, die erhaltenen diastereoisomeren Salze durch fraktionierte Kristallisation auftrennt und anschliessend die optisch einheitlichen Basen freisetzt.

3. Verfahren nach Anspruch 1 oder 2, dadurch gekennzeichnet, dass die Umsetzung in einem Gemisch von Methanol und Methylenchlorid erfolgt.

4. Salz von (−)-2,2′-(1,1′-Binaphthyl)phosphorsäure mit (+)-3-(1-Propyl-3-piperidinyl)phenol.

5. Salz von (+)-2,2′-(1,1′-Binaphthyl)phosphorsäure mit (−)-3-(1-Propyl-3-piperidinyl)phenol.

6. Salz von (−)-2,2′-(1,1′-Binaphthyl)phosphorsäure mit (+)-3-(3-Methoxyphenyl)-1-propylpiperidin.

7. Salz von (+)-2,2′-(1,1′-Binaphthyl)phosphorsäure mit (−)-3-(3-Methoxyphenyl)-1-propylpiperidin.

**Claims**

1. A process for the manufacture of the two enantiomers of 3-(1-propyl-3-piperidinyl)phenol of the formula

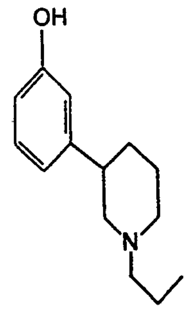

I

characterized by resolving a racemic compound of the general formula

(±)

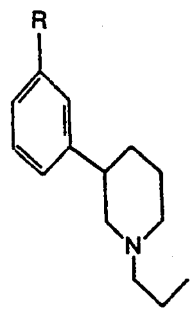

V

wherein R signifies hydroxy or methoxy, with optically active 2,2′-(1,1′-binaphthyl)phosphoric acid into the two enantiomers and, if necessary, cleaving the methoxy group in the 3-position.

2. A process according to claim 1, characterized in that racemic 3-(1-propyl-3-piperidinyl)phenol is reacted with optically active 2,2′-(1,1′-binaphthyl)phosphoric acid, the diastereoisomeric salts obtained are separated by fractional crystallization and subsequently the optically uniform bases are liberated.

3. A process according to claim 1 or 2, characterized in that the reaction is carried out in a mixture of methanol and methylene chloride.

4. The salt of (−)-2,2′-(1,1′-binaphthyl)phosphoric acid with (+)-3-(1-propyl-3-piperidinyl)-phenol.

5. The salt of (+)-2,2′-(1,1′-binaphthyl)phosphoric acid with (−)-3-(1-propyl-3-piperidinyl)-phenol.

6. The salt of (−)-2,2′-(1,1′-binaphthyl)phosphoric acid with (+)-3-(3-methoxyphenyl)-1-propylpiperidine.

7. The salt of (+)-2,2′-(1,1′-binaphthyl)phosphoric acid with (−)-3-(3-methoxyphenyl)-1-propylpiperidine.

**Revendications**

1. Procédé de préparation des deux énantiomères du 3-(1-propyl-3-pipéridinyl)-phénol de formule

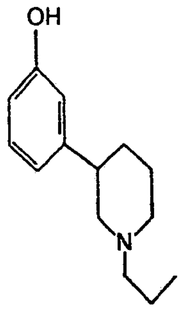

I

caractérisé en ce que l'on sépare un composé racémique de formule générale

(±)

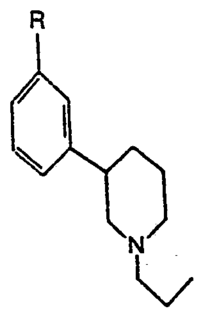

V

dans laquelle R représente un groupe hydroxy ou méthoxy, en les deux énantiomères à l'aide de l'acide 2,2'-(1,1'-binaphtyl)-phosphorique optiquement actif, et lorsque c'est nécessaire, on élimine le groupe méthoxy en position 3.

2. Procédé selon la revendication 1, caractérisé en ce que l'on fait réagir le 3-(1-propyl-3-pipéridinyl)-phénol racémique avec l'acide 2,2'-(1,1'-binaphtyl)-phosphorique optiquement actif, on sépare les sels diastéréoisomères obtenus par cristallisation fractionnée puis on libère les bases à l'état d'isomères optiquement purs.

3. Procédé selon la revendication 1 ou 2, caractérisé en ce que la réaction est effectuée dans un mélange de méthanol et de chlorure de méthylène.

4. Le sel de l'acide (−)-2,2'-(1,1'-binaphtyl)-phosphorique et du (+)-3-(1-propyl-3-pipéridinyl)-phénol.

5. Le sel de l'acide (+)-2,2'-(1,1'-binaphtyl)-phosphorique et du (−)-3-(1-propyl-3-pipéridinyl)-phénol.

6. Le sel de l'acide (−)-2,2'-(1,1'-binaphtyl)-phosphorique et de la (+)-3-(3-méthoxyphényl)-1-propylpipéridine.

7. Le sel de l'acide (+)-2,2'-(1,1'-binaphtyl)-phosphorique et de la (−)-3-(3-méthoxyphényl)-1-propylpipéridine.